# EUROPEAN PATENT APPLICATION

(11) **EP 3 582 228 A1**
(43) Date of publication of application: **18.12.2019**
(21) Application number: 18177220.3
(22) Date of filing: 12.06.2018
(51) Int. Cl.: G16H 40/20, G16H 50/20

(54) **METHOD, SYSTEM AND MOBILE COMMUNICATIONS DEVICE MEDICAL FOR OPTIMIZING CLINICAL CARE DELIVERY**

(71) Applicant: Bittium Biosignals Oy, 70800 Kuopio (FI)
(72) Inventor: Sarén, Matti, 87250 Kajaani (FI)
(74) Representative: LEITZINGER OY

(57) **Abstract**

The present disclosure describes a method and a system implementing the method for optimizing scheduling of clinical care delivery of a patient and work flow of medical personnel. The method comprises determining a current value of at least one condition indicator of the patient on the basis of at least one biosignal of the patient, said at least one biosional originating from at least one sensor configured to automatically measure said at least one biosignal, forming a prediction of a clinical care delivery need of the patient on the basis of the current value and at least one preceding value of the at least one condition indicator, and updating scheduling of the patient's clinical care delivery on the basis of the determined prediction.

## Description

### FIELD

The invention relates to clinical care, and in particular, to systems for optimizing clinical care delivery.

### BACKGROUND INFORMATION

When a patient checks in for clinical care, his or her condition may first be assessed. The assessment may include measurement of various biosignals of the patient. Based on the results of the assessment, a care plan may be formed. The care plan typically determines a schedule for clinical care delivery, e.g. the suitable interval of check-ups on the patient's condition and administering of medicine to the patient. The patient may then monitored and treated according to the plan. Vital functions (heart and respiratory system) of the patient may be monitored during the care, and an alarm may be raised if an event in the vital functions is detected.

Traditionally, a doctor in charge of tending a patient determines the care plan for the patient. However, there may be only a very limited time to get familiar with the patient history and the symptoms. Further, evaluation of the risks and the suitable treatment depends greatly on the doctors' expertise. It may therefore be challenging to ensure consistency and quality of the decisions. Further, the condition of a patient may not be a static. The condition may change very quickly, and therefore, there may be a need for adjusting the scheduling of the clinical care delivery accordingly.

### BRIEF DISCLOSURE

An object of the present disclosure is to provide a method for analysing medical data and a system for implementing the method so as to alleviate the above disadvantages. The object of the disclosure is achieved by a scheduling method and a system which are characterized by what is stated in the independent claims. The preferred embodiments of the disclosure are disclosed in the dependent claims.

In a system according to the present disclosure, a patient checking in for clinical care may be equipped with a sensor or sensors that automatically measure at least one biosignal of the patient. The system then suggests an initial care plan for the patient by using measurement data obtained with the sensors. Medical personnel may review the initial care plan and make adjustments to it when necessary. The system may use a calculation model or models to determine and show correlations between detectable trends (and/or changes of trends) in biosignals and a suitable scheduling of care delivery. Machine learning may be utilized in forming these models, for example. The adjustments to the care plans made by medical personnel may be utilized in improving the models.

During the execution of the care plan, the system may continuously update the care plan based on new measurement data from the sensors monitoring the patient. The system may inform medical personnel about changes to the care plan via mobile communications devices, such as a smart phone or a tablet computer, carried by the medical personnel.

The method and system according to the present disclosure provide new, reliable means for monitoring the condition of a patient. Further, with the method and system, the scheduling of clinical care delivery of a patient may be automatically adjusted. Safety, consistency, and quality of care of the patient can be improved without significantly increasing the workload of the medical personnel.

### BRIEF DESCRIPTION OF THE DRAWINGS

In the following the invention will be described in greater detail by means of preferred embodiments with reference to the attached drawings, in which
Figure 1 shows a simplified example of a system according to the present disclosure; and
Figure 2 shows another simplified example of a system according to the present disclosure.

### DETAILED DISCLOSURE

The present disclosure describes a method for optimizing scheduling of healthcare delivery, such as scheduling of clinical care delivery in a healthcare facility, e.g. a hospital. In the context of the present disclosure, healthcare/clinical care delivery may be understood as assignments performed by medical personnel in order to deliver clinical care to a patient. The clinical care may take place in a healthcare facility, such as a hospital. In addition treatments, clinical care delivery comprises also other aspects, such as check-ups on the patient's condition by medical personnel. Thus, scheduling of healthcare delivery can also be understood as scheduling of work flow of medical personnel.

In a method according to the present disclosure, at least one biosignal of the patient is measured. At least one sensor may be configured to automatically measure the at least one biosignal of the patient. In the context of the present disclosure, a biosignal may be a signal that can be consistently measured and monitored in a human body. A biosignal may be electrical or non-electrical. Electroencephalography (EEG) and electrocardiography (ECG) are examples of electrical biosignals. Peripheral oxygen saturation (SpO₂) is an example of non-electrical biosignal.

The measured biosignal data originating from the at least one sensor may be used for determining a current value of at least one condition indicator of the patient. A condition indicator may represent an aspect of a patient's condition (e.g. level of consciousness) that can be derived from the biosignal data. A condition indicator may represent a biosignal as such, a feature of a biosignal (e.g. statistical or spectral feature), or a computational function of a plurality of biosignals and/or their features. Biosignal data representing the at least one biosignal is preferably in the form of real-time or near real-time measurement samples so that the value of the condition indicator represents actual, current condition of the patient.

The condition indicators may be used to determine an overall condition score representing the degree of illness of a patient, similar to an Early Warning Score (EWS), for example. However, there is a distinct difference between Early Warning Scores and the condition score in a method according to the present disclosure. In EWS, each measured signal has fixed trigger limits. Each biosignal is individually categorized based on the trigger limits and the patient's condition is determined based on the categorized biosignals. In contrast in the method according to the present disclosure, the estimate on the patient's condition may be based on trends detectable in the condition indicators, rather than mere trigger limits on individual biosignals.

The trends may be in the form patterns that can be detected in the biosignal data. Thus, a prediction of a clinical care delivery need of the patient may be formed based on at least one pattern detectable in the at least one condition indicator, and the scheduling of the patient's clinical care delivery may be updated on the basis of the determined prediction. The at least one pattern may be detected on the basis of the current value and at least one preceding value of the at least one condition indicator. In a simple embodiment, the pattern may be a detectable change in the at least one condition indicator within a predetermined time period, the time period being ranging from seconds to hours, e.g. 15 seconds, 5 minutes or 1 hour.

In order to form a prediction, a method according to the present disclosure may comprise forming a predictive model on patient's care delivery need. The prediction of the clinical care delivery need may then be determined on the basis of the predictive model. The predictive model may be configured to detect patterns in the condition indicators, and to produce the condition score based on the condition indicators. Machine learning may be utilized in forming the model, for example. With machine learning, correlations between the degree/severity of illness and patterns in condition indicators can be found and utilized. Based on the condition score, the urgency of care can be estimated. In addition to biosignal data, a model according to the present disclosure may take into account also other parameters, such as age, gender, and weight of the patient. In this manner, models for specific groups may be formed.

In some embodiments, the model may directly produce an estimate of the urgency of care delivery instead of a condition score. The model may even directly produce a suggestion for a timing value for scheduling of the clinical care delivery. For example, the model may determine a timing value that acts as a direct estimate of optimal interval for check-ups on the patient on the basis of the at least one measured biosignal. Feedback from the medical personnel us may be utilized to improve the model. For example, when the model provides an initial suggestion for the value of the condition score and/or for the timing value of the scheduling of the clinical care delivery, the medical personnel may confirm the correctness of the suggestions, and if the suggestions are not correct, adjust the suggested values. The confirmations and adjustments may then be used as an input for adjusting the predictive model.

In order to optimize clinical care delivery of a patient, a system according to the present disclosure may be used. The system comprises one or more analytics units, each of which can provide a suggestion for optimizing the scheduling of a patient's clinical care delivery. Thus, the analytics unit can also act as a unit for optimizing work flow of medical personnel in a healthcare facility.

The analytics unit may comprise calculating means configured to receive biosignal data of at least one biosignal of at least one patient, determine a current value of a condition indicator on the basis of the biosignal data, determine a prediction of a clinical care delivery need of the patient on the basis of the current value and at least one preceding value of the condition indicator, and update scheduling of the clinical care delivery on the basis of the determined prediction.

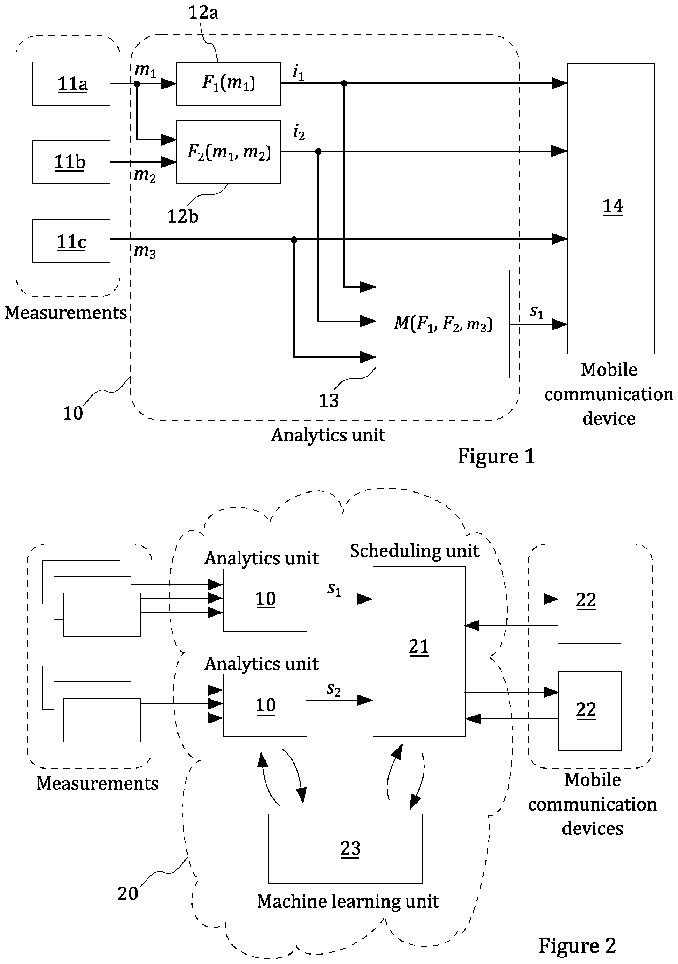

Figure 1 shows a simplified example of a system according to the present disclosure. In Figure, the system comprises an analytics unit 10 that is configured to receive measured biosignal data *m*₁ to *m*₃ from measurement sensors 11a to 11c attached to a patient. The analytics unit 10 comprises preprocessing units 12a and 12b in Figure 1. The preprocessing units may be configured to extract a feature from one or more of the biosignal data *m*₁ to *m*₃. The extracted feature then acts as a condition indicator. For example, in Figure 1, preprocessor 12a performs a function *F*₁ on biosignal data *m*₁ in order to extract condition indicator *i*₁, and preprocessor 12b performs a function *F*₂ on measurement data *m*₁ and *m*₂ in order to extract condition indicator *i*₁. Further, in Figure 1, biosignal data *m*₃ acts as a condition indicator without preprocessing.

The analytics unit 10 in Figure 1 further comprises a predictive model 13. The predictive model 13 that is in the form of algorithm *M* in Figure 1 calculates by a predictive scheduling data *s*₁ based on the condition indicators. The scheduling data *s*₁ contains information for updating the scheduling of clinical care delivery of the patient. For example, the scheduling data may be in the form of a condition score representing the degree of illness of the patient, thereby indicating the urgency of clinical care delivery. Alternatively, or in addition, the scheduling data may comprise a suggestion for timing of the clinical care delivery, e.g. in the form of a suggested interval for check-ups. The preprocessing units 12a to 12b and the predictive model 13 may be implemented on a computing device, such as a computer, computer server cluster, or a computing cloud can serve as the calculating means, for example.

The scheduling data *s*₁ can be used for optimizing the scheduling of clinical care delivery of a patient and/or the assignments of medical personnel. In Figure 1, the system further comprises a communications device 14 carried by a member of medical personnel. The mobile communications device 14 may a smart phone or a tablet computer, carried by a nurse or a doctor tending the patient, for example. The analytics unit provides the scheduling data *s*₁ to the mobile communications device 14. The mobile communications device 14 may be configured to display a list of upcoming work assignments of said member of medical personnel. Based on the predictive scheduling data si, the device 14 may update the list and provide a suggestion of the order of the tasks. For example, if the scheduling data indicates that the patient is in need of urgent clinical care, the device 14 may update the work assignments accordingly. The patient's care plan may be updated such that a priority value given to the task of monitoring the patient becomes higher, and the interval of the monitoring task becomes shorter.

The device 14 may receive scheduling data from a plurality of analytics units 13, each analytics unit 13 being assigned to an individual patient. The device 14 may act as a scheduling unit for the member of medical personnel, and all work assignments in the list of upcoming work assignments may be scheduled (and rescheduled) automatically based in the scheduling data from the analytics units. As also shown in Figure 1, the analytics unit 13 may provide the condition indicators to the mobile communications device 14. The device 14 may display a dashboard of vital functions of the patients based on the measured biosignal data and/or one or more of the t condition indicators, thereby enabling the medical personnel to easily assess the condition of the patient.

A system according to the present disclosure may also be used for optimizing scheduling of clinical care delivery of a plurality of patients and/or optimizing scheduling of work assignments of a plurality of medical personnel. An analytics unit according to the present disclosure may provide the scheduling data *s*₁ to a shared scheduling unit that controls scheduling of work assignments of a plurality of medical personnel. Figure 2 shows another simplified example of a system according to the present disclosure.

In Figure 2, the system further comprises a scheduling unit 21 that receives scheduling data *s*₁ from analytics units 10 of a plurality of patients. The analytics units 10 may be similar or the same as in Figure 1, for example. The scheduling unit 21 may hold schedules of work assignments of a plurality of medical personnel, and adjust the assignments according to scheduling data from the analytics units 10. For example, if an analytics unit 10 indicates that a patient has an urgent need, the scheduling unit may adjust the assignment schedule of a member of medical personnel tending the patient or create a new assignment to a different member of the medical personnel.

In the system of Figure 2, members of medical personnel carry mobile communications devices 22 configured to receive assignment information from the scheduling unit 21. Based on the assignment information, the communications devices 22 show a list of upcoming work assignments. Preferably, the assignment information also provides a suggestion of the order in which the assignment may be performed. The scheduling unit 21 and the analytics units 10 may be implemented at various computing platforms, such as single computer, computer clusters or computing clouds. In Figure 2, the scheduling unit 21 and the analytics units 10 are implemented on a computing cloud 20.

The system in Figure 2 also comprises a machine learning unit 23 within the computing cloud 20. The machine learning unit 23 may be configured to receive biosignal data and/or condition indicator data from a plurality of patients. Based on this data, the machine learning unit 23 may search for new correlations between measurement data and needs for clinical care delivery. In response, the machine learning unit 23 may provide the analytics units 10 with improved parameters for their predictive models 13, for example.

In addition to biosignal data and the condition indicators, the machine learning unit 23 may receive feedback data from the medical personnel. The mobile communications devices 22 may implement an interface for user input, e.g. in the form of confirmations buttons for each work assignment on graphical user interfaces of the devices 22, for example. When an assignment has been successfully finished, medical perform may provide input confirming that the assignment has been done. The devices 22 then transmit the confirmations to the scheduling unit 21 and/or the machine learning unit 23. Based on the feedback provided by the medical personnel, the scheduling unit 21 may update the schedules of the medical personnel. The feedback also provides information for machine learning unit 23 on how well the intervals for care delivery suggested by the predictive models correspond with actual intervals realized in the healthcare facility.

Alternatively, or in addition, geolocation capabilities (when present) of the mobile communications device may be utilized in confirming the completion of an assignment. Further, the system may comprise one or more identifier devices that may be used for providing feedback from the medical personnel. The identifier device may be a QR code, a NFC tag or a Bluetooth beacon attached to a wrist band and/or bed of the patient, for example. Members of medical personnel may use the mobile communications devices 22 together with the identifier devices to confirm completion of work assignments. In addition, the identifier devices may be configured to provide other information (e.g. information related to patient's status) in order to help the medical personnel to make right decisions.

In some embodiments, the system (e.g. a machine learning unit) may be configured provide suggestions of optimal routes for hospital rounds made by the medical personnel. For example, the system may gather information time delays between timestamps between two rooms, and form an estimate of an effective distance between two rooms. The effective distance may represent a walking distance between the rooms, including any hindrances (e.g. doors, elevators, stairs). When the system has estimates of effective distances between rooms, it may form a model of the network of rooms based on the distances. An optimal route for a hospital round may then be estimated based on the model of the network of rooms and the priority of the tasks. A heuristic algorithm, similar to algorithms intended for solving the travelling salesman problem, may be used, for example.

The above-described method and system is now discussed in the form of an exemplary embodiment. First, a patient may be provided with sensors measuring biosignals of the patients. The sensors may be wireless sensors that are able to transmit their measurements in real-time or near real-time. The sensors may be configured to measure EEG, ECG, oxygen saturation, temperature, and blood pressure, for example.

One or more analytics units according to the present disclosure may be configured to form at least one condition indicator of the patient based on the measured raw biosignal data. For example, an analytics unit may be configured to determine level of consciousness based on EEG data. Further, one or more analytics units may produce condition indicators that indicating various heart arrhythmias based on ECG data. The analytics units may be implemented in a computing cloud, for example.

The condition indicators (and in some embodiments also the raw biosignal data) may be inputted to a predictive model. The predictive model then determines the need (urgency) of clinical care delivery of the patient. The scheduling of the clinical care delivery of the patient (e.g. check-ups), and thus the scheduling of medical personnel tending the patient, may then be updated on the basis of the determined need. The predictive model may also directly alarm the medical personnel if a patient is in an urgent need of clinical care. In addition to the predictive model, alarms based on monitoring vital signs of the patient may also be implemented.

Schedules of the medical personnel may be displayed on displays of mobile communications devices carried by the personnel. The mobile communications devices may be smart phones with a scheduling application, for example. The scheduling application may be configured to display a list of work assignments in an optimal order. Further, the scheduling application may display graphs/trends of the condition indicators / biosignals for each assignment.

It is obvious to a person skilled in the art that the electrode patch and the detection method/system can be implemented in various ways. The invention and its embodiments are not limited to the examples described above but may vary within the scope of the claims.

## Claims

1. A method for optimizing scheduling of clinical care delivery of a patient, wherein method comprises
determining a current value of at least one condition indicator of the patient on the basis of at least one biosignal of the patient, said at least one biosional originating from at least one sensor configured to automatically measure said at least one biosignal,
forming a prediction of a clinical care delivery need of the patient on the basis of the current value and at least one preceding value of the at least one condition indicator, and
updating scheduling of the patient's clinical care delivery on the basis of the determined prediction.

2. A method according to claim 1, wherein the method comprises determining the prediction of the clinical care delivery need of the patient on the basis of a predictive model of the patient's care delivery need, wherein the predictive model indicates a correlation between the clinical care delivery need and a pattern detectable in the at least one condition indicator.

3. A method according to any one of preceding claims, wherein the updating of scheduling of the clinical care comprises changing an interval of check-ups on the patient's condition by medical personnel.

4. A method according to any one of preceding claims, wherein the method comprises displaying the scheduling on a mobile communications device.

5. An analytics unit comprising means configured to
receive biosignal data of at least one biosignal of a patient,
determine a current value of a condition indicator of the patient on the basis of the biosignal data,
determine a prediction of a clinical care delivery need of the patient on the basis of the current value and at least one preceding value of the condition indicator, and
update scheduling of the clinical care delivery on the basis of the determined prediction.

6. An analytics unit to claim 5, wherein the analytics unit comprises
a predictive model of the patient's care delivery need, wherein the predictive model indicates a correlation between the clinical care delivery need and a pattern detectable in the at least one condition indicator.

7. A mobile communications device comprising means configured to
receive information on a patient's clinical care delivery, wherein the information represents scheduling determined with a method according to claim 1, and
display the scheduling on a mobile communications device.
